(19) Europäisches Patentamt
European Patent Office
Office européen des brevets

(11) Publication number: **0 124 186 B1**

(12) **EUROPEAN PATENT SPECIFICATION**

(45) Date of publication of patent specification: **21.09.88**

(51) Int. Cl.⁴: **C 07 D 233/60**, C 07 D 401/12, C 07 D 409/12, A 61 K 31/415

(21) Application number: **84200621.5**

(22) Date of filing: **02.05.84**

# (54) Imidazole-ethanol esters.

(30) Priority: **03.05.83 NL 8301550**

(43) Date of publication of application: **07.11.84 Bulletin 84/45**

(45) Publication of the grant of the patent: **21.09.88 Bulletin 88/38**

(84) Designated Contracting States: **AT BE CH DE FR GB IT LI LU NL SE**

(56) References cited:
**EP-A-0 082 982**
**DE-A-3 122 175**
**FR-A-1 486 817**
**SU-A- 380 652**

**CHEMICAL ABSTRACTS, Vol. 83, NO. 13, 29.09.1975, COLUMBUS, OHIO (US), Page 602, column 1, abstract nr 114.815p**

**CHEMICAL ABSTRACTS; Vol. 96, No. 23, June 7, 1982, COLUMBUS, OHIO (US), page 677, column 2, abstract nr. 199.719b**

(73) Proprietor: **GIST-BROCADES N.V.
Wateringseweg 1 P.O. Box 1
NL-2600 MA Delft (NL)**

(72) Inventor: **Hofman, Petrus Simon
Mr. J. Gerritszlaan 63/2
NL-2024 KL Haarlem (NL)**
Inventor: **Hall, David William Reid
Grindweg 147
NL-3054 VJ Rotterdam (NL)**
Inventor: **Jaitly, Kapil Dev
Henri Dunantlaan 99
NL-2614 GL Delft (NL)**

(74) Representative: **Schmieman, Johannes Hendrik et al
Gist-Brocades N.V. Patents & Trademarks Department Martinus Nijhofflaan 2 P.O. Box 1
NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 124 186 B1

**Description**

The invention relates to 1H-imidazole-1-ethanol and esters derived therefrom as sebumsynthesis-inhibiting agents, pharmaceutical preparations comprising these compounds and their preparation and also to new 1H-imidazole-1-ethanol esters and their preparation.

1H-imidazole-1-ethanol is a known compound, inter alia mentioned in British patent 939681. Various patents and patent applications relate to imidazolederivatives, includng 1H-imidazole-1-ethanol esters. There can be mentioned French patent 1486817, German patent application 2509473 and European patent 19359. In these patents the esters are not specifically described. The acetate is known from J. Am. Chem. Soc. *100* (11), 3575 (1978). Applications mentioned of these compounds are insecticides, bitumen improvers, herbicides and intermediates. A therapeutic activity is nowhere mentioned.

It has now been found that 1*H*-imidazole-1-ethanol and a group of esters thereof show an interesting therapeutic activity. The esters are the compounds of the general formula

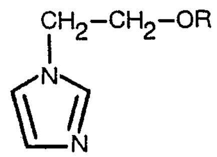

I

in which R represents an acyl residue of an organic carboxylic acid which does not bear further carboxylic groups, either in the free or salt form, and their acid addition salts.

The group R can be derived from a diversity of acids; for instance alkanecarboxylic acids such as acetic acid, butanoic acid and hexadecanoic acid; benzoic acid and substituted benzoic acids; heterocyclic acids such as 4-pyridinecarboxylic acid and 2-thiophene carboxylic acid; cinnamic acid and retinoic acid. The imidazole derivatives according to formula I show very good reversible local sebum synthesis inhibition. As the compounds penetrate very well in the skin and mostly give little irritation they are particularly suited for the therapeutic treatment of acne and seborrhoe. The activity of the imidazole derivatives is specifically directed to the sebum synthesis; they have no effect on the lipid synthesis in other organs than the sebaceous glands.

Up to now no compounds are known with such a specific activity. There are sebum synthesis inhibitors for oral application: cyproteron acetate and isotretinoin. The oral application is more prone to side effects than local application. Due to its anti-androgenic properties cyproteron acetate is only applicable in women. Isotretinoin has so many detrimental side effects, that it is only used in very persistent, serious cases of acne. The new imidazole derivatives according to the invention are therefore an important therapeutic gain.

Esters of 1H-imidazole-1-ethanol with a great variety of acids prove all to inhibit the sebum synthesis. Compounds to be mentioned particularly as having a good activity are

a) esters with benzoic acid, which may be substituted with lower alkyl or alkoxy groups or chlorine, bromine or iodine atoms, particularly the benzoic acid ester;

b) esters with phenylalkanecarboxylic acids, in which the phenyl group may be substituted with lower alkyl or alkoxy groups or chlorine, bromine or iodine atoms and in which the alkane part has at most 4 carbon atoms, particularly the benzenepropanoic acid ester;

c) esters with acids of the formula ACOOH, in which A represents a sulphur, oxygen or nitrogen containing heterocyclic five- or six-membered ring, particularly the 2-thiophenecarboxylic acid ester;

d) esters with aliphatic carboxylic acids, particularly the 2,2-dimethylpropanoic acid ester.

The term "lower" is used here to indicate alkyl or alkoxy-groups with at most 6 carbon atoms.

The 2-(1*H*-imidazol-1-yl)ethyl benzoate is particularly preferred. It gives in human a good inhibition of the sebum secretion, a good penetration of the skin and little irritation. In animal tests no lipid synthesis inhibition in other organs or hormonal activity were found.

The sebum synthesis inhibiting activities of 1*H*-imidazole-1-ethanol and the esters of formula I were established in a number of animal species. Particularly in the hamster much research has been carried out. Androgens administered to female hamsters stimulate the sebum synthesis in ear sebaceous glands. The extent of sebum synthesis is determined by incubating earbiopsies with radioactive acetate (Hall D.W.R. et al, Hormonal Control of Hamster Ear Sebaceous Gland Lipogenesis, Arch. Dermatol Res, *275*, 1 (1983)). The test is based on the conversion of the added radioactive acetate into lipids which after extraction with hexane can be determined in a scintillation counter. In the following table the inhibition percentages of a number of substances are shown. The percentages may rise over 100%. A very strong activity of a compound will namely inhibit both the activity stimulated by androgen and the basal sebum synthesis activity of the sebaceous gland.

| R'*) | salt/base | doses (µg per day) | lipid synthesis inhibition (%) |
|---|---|---|---|
| 2,4-$Cl_2C_6H_3$- | maleate | 100 | 37 |
| ditto | base | 100 | 60 |
| 4-$ClC_6H_4$- | maleate | 500 | 49 |
| 4-$CF_3C_6H_4$- | maleate | 500 | 70 |
| 4-$tBuC_6H_4$- | HCl | 500 | 81 |
| ditto | base | 100 | 67 |
| 4-$CH_3C_6H_4$- | maleate | 500 | 92 |
| ditto | base | 100 | 91 |
| 4-$CH_3OC_6H_4$- | base | 100 | 83 |
| $C_6H_5$- | base | 100 | 82 |
| $CH_3(CH_2)_2$- | base | 100 | 106 |
| $CH_3$- | base | 100 | 82 |
| $C_6H_5(CH_2)_2$- | base | 100 | 76 |
| 4-pyridyl- | oxalate | 100 | 44 |
| $C_6H_5$-CH=CH- | base | 100 | 59 |
| $(CH_3)_3$-C- | base | 100 | 72 |
| $CH_3(CH_2)_{14}$- | oxalate | 100 | 44 |
| 2-thiophene- | base | 100 | 89 |
| retinoic acid (=R'COOH) | oxalate | 100 | 39 |
| imidazolethanol | Maleate | 1000 | 37 |

*) R'CO = R

1*H*-imidazole-1-ethanol and the compounds according to formula I can be applied as the free base or in the form of a pharmacological acceptable acid addition salt. The term "pharmacological acceptable" means here that at effective doses the salt does not show unacceptable side-effects.

The compounds can be applied topically in the form of gels, creams, lotions and the like.

Preferably, a dosage form is used which comprises 0.5—10% by weight of the active agent. The invention relates to 1H-imidazole-1-ethanol and the esters of formula I as sebum synthesis inhibitors. The invention further includes pharmaceutical preparations containing these compounds and their preparation. The compounds of formula I in which R is as hereinbefore defined with the exception of the acetic acid residue are new. Said compounds and their acid addition salts as such and their preparation are also a feature of the invention.

The compounds according to formula I may be prepared by methods known for analogous compounds. For instance 1*H*-imidazole-1-ethanol may be reacted with an acid halide derived from R. The reaction is preferably carried out by heating the reactants in a polar solvent, such as acetonitrile, methyl isobutyl ketone or dioxane, in the presence of an acid binding agent such as trimethylamine.

The compounds can also be prepared by reacting imidazole with a compound of the formula

$$X—CH_2—CH_2—OR \qquad II$$

in which R is as hereinbefore defined and X represents a halogen atom (preferably chlorine, bromine or iodine). Preferably the two reactants are heated together without a solvent. Compounds of formula II may be prepared by reacting the appropriate acid (ROH) with a compound of the formula

$$X—CH_2—CH_2—OH \qquad III$$

in which X is as hereinbefore defined.

The preparation of the imidazole-ethanol ester of formula I is illustrated by the following examples. The structure of the compounds prepared was confirmed by the IR- and NMR-spectra.

Example 1

11 g (0.05 mol) of 2,4-dichlorobenzoyl chloride were added dropwise to a mixture of 5.5 g (0.05 mol) of 1*H*-imidazole-1-ethanol[1]), 20 ml of triethylamine and 150 ml of acetonitrile. The reaction mixture was kept at ca. 80°C for another 1.5 hours and then the solvent was evaporated. The residue was dissolved in a mixture of ethyl acetate and diethyl ether and it was then extracted with water and subsequently with dilute hydrochloric acid. The acidic aqueous layer was made alkaline, after which the base was taken up in ethyl acetate. The organic layer was extracted three times with water, dried and partly concentrated by evaporation. The residue was acidified with maleic acid and the 2-(1*H*-imidazol-1-yl)ethyl, 2,4-dichlorobenzoate (*Z*)-2-butenedioate (1:1) obtained was crystallized from a mixture of 2-propanol, acetone and diethyl ether.

Melting point 116—117°C.

With the aid of sodium bicarbonate the base was liberated from the salt.

Melting point 80.5°C.

1) prepared from imidazole and ethylene carbonate according to British patent 939.681.

Example 2

By a similar method as described in Example 1 the following compounds of formula I were prepared.

| RO | solvent | salt | melting point (°C) |
|---|---|---|---|
| $4-CF_3-C_6H_4-COO$ | acetonitrile | maleate | 106-107 |
| $4-Me-C_6H_4-COO$ | acetonitrile | maleate | 103-104 |
| $4-Me-C_6H_4-COO$ | acetonitrile | base[1]) | oil |
| $4-tBu-C_6H_4-COO$ | acetonitrile | HCl | 104-105 |
| $4-tBu-C_6H_4-COO$ | acetonitrile | base[3]) | oil |
| $4-Cl-C_6H_4-COO$ | acetonitrile | maleate | 137-138 |
| $CH_3-COO$ | dioxane | base[2]) | oil |
| $C_3H_7-COO$ | dioxane | base[2]) | oil |
| $4-MeO-C_6H_4-COO$ | acetonitrile | base[3]) | oil |
| $C_6H_5C_2H_4-COO$ | dioxane | base | ca. 30 |
| $4-C_5NH_4-COO$ | dioxane | ½oxalate[4]) | 159-160 |
| $C_6H_5CH=CH-COO$ | dioxane | base[5]) | 109-110 |
| $(CH_3)_3-COO$ | dioxane | base[2]) | oil |

1) via maleate

2) purified with the aid of column chromatography (silicagel; ethyl acetate/methanol/trimethylamine 100:40:1)

3) via hydrochloride

4) salt crystallized from a mixture of methanol and ethanol

5) crystallized from a mixture of methanol and acetone.

Example 3

13.8 g (0.1 mol) of salicylic acid were added to a mixture of 80 ml of thionylchloride and ca. 1 g of aluminium chloride at 30—40°C. The mixture was then stirred at 45°C for 2 hours and it was then concentrated by evaporation.

The solution of the acid chloride obtained in 40 ml of dioxane was added dropwise to a mixture of 11.2 g (0.1 mol) of 1*H*-imidazole-1-ethanol, 40 ml of triethylamine and 50 ml of dioxane. The reaction mixture was refluxed for 1.5 hour and it was then concentrated by evaporation. The residue was dissolved in ethyl acetate and the solution was shaken with dilute hydrochloric acid. The acidic aqueous layer was made alkaline and the base separated off was taken up in a mixture of diethyl ether and ethyl acetate.

The organic solution was then dried and concentrated by evaporation. Finally, the solid residue was crystallized in a mixture of toluene and petroleum ether (boiling range 28—40°C). The 2-(1*H*-imidazol-1-yl)ethyl 2-hydroxybenzoate had a melting point of 74—75°C.

Example 4

In a similar way as described in Example 3, but with substitution of the salicylic acid by an equivalent of palmitic acid and conversion in acetone of the base obtained into the oxalate, the 2-(1*H*-imidazol-1-yl)ethyl hexadecanoate ethanedioate (1:1) was obtained.

Melting point 99—100°C.

Example 5

A warm solution of 220 g (2.5 mol) of ethylene carbonate and 1 g of 4-toluenesulphonic acid in 200 ml of methyl isobutyl ketone (MIK) was added in about 60 minutes to a refluxing solution of 136 g (2 mol) of imidazole in 200 ml of MIK. The mixture was then refluxed for another 2 hours. After cooling, 300 ml of triethylamine were added. To the mixture obtained 280 g (231 ml, 2 mol) of benzoyl chloride were subsequently added dropwise in 45 minutes at 40—100°C, after which the mixture was stirred at 100—110°C for another 2 hours. Subsequently the reaction mixture was cooled to ca. 10°C. The solid substance obtained was sucked off and washed with ca. 250 ml of MIK. The filtrate was concentrated by evaporation and the residue was dissolved in dichloromethane.

Successively the solution was washed three times with water, dried, decoloured with some silicagel, filtered and concentrated by evaporation. 365 g of 2-(1*H*-imidazol-1-yl)ethyl benzoate were obtained which without dilution were acidified with a warm saturated solution of maleic acid in acetone. After cooling to −10°C, the maleate was sucked off and washed with acetone and subsequently dried at about 80°C in vacuo. Yield 365 g.

The mother lye was concentrated by evaporation and the residue was converted into the base, which subsequently was taken up in dichloromethane. The solution was washed, dried, decoloured, filtered and concentrated by evaporation. The residue was converted again in the maleate. 52 g were obtained.

Both portions of maleate were together converted into the base, which was taken up in dichloromethane, after which the solvent was evaporated again. Melting point 52—53°C Boiling point 174°C/0.5 mm Hg (67 Pa).

Example 6

A mixture of 75 g of mandelic acid, 80 g of 2-chloro-ethanol, 100 ml of benzene, 100 ml of petroleumether (60—80°C) and some p-toluenesulphonic acid was refluxed under removal of the water formed. After completion of the reaction the mixture was concentrated by evaporation, after which the residue was taken up in diethyl ether. The ethereal solution was washed with alkaline water, dried and concentrated by evaporation. The chloro-ethylester of mandelic acid was obtained. A mixture of 0.1 mol of this ester and 0.2 mol of imidazole was heated for 5 min. at 130—150°C. After cooling, water and diethyl ether were added to the mixture. The ethereal layer was separated off, washed with water and then extracted with dilute hydrochloric acid. Subsequently, the base was liberated from the acidic aqueous layer with the aid of dilute lye. This was taken up in diethylether and converted into the oxalate, which finally was crystallized from a mixture of ethanol and diethyl ether. 2-(1*H*-imidazol-1-yl)ethyl alpha-hydroxybenzeneacetate ethanedioate (2:1) was obtained with melting point 131—132°C.

Example 7

In a similar way as described in Example 3, 2-(1*H*-imidazol-1-yl)ethyl 2-thiophenecarboxylate was obtained from 1*H*-imidazole-1-ethanol and 2-thiophenecarboxylic acid chloride in the form of an oil. The base was purified with the aid of column chromatography (silicagel, eluent dichloromethane/ethanol 95:5).

Example 8

To a suspension of 12 g (0.04 mol) of retinoic acid (vitamin A acid) and 3.4 ml of pyridine in 160 ml of diethyl ether there were added dropwise at −5°C 3.25 ml of thionyl chloride dissolved in 20 ml of diethyl ether. The mixture was stirred for another 2 hours at 0°C, after which it was filtered. The solid substance was washed with diethyl ether. The filtrate was then added dropwise at 0°C to a solution of 5 g of 1*H*-imidazole-1-ethanol and 10 ml of triethylamine in circa 100 ml of dioxane. The reaction mixture was stirred for another 2 hours at 20°C and it was then filtered and concentrated by evaporation. The residue was taken up in dichloromethane, after which the organic solution was washed with water, with a solution of sodium bicarbonate in water and finally again with water and subsequently it was dried and concentrated by evaporation. The solution of the base obtained in diethyl ether was acidified with oxalic acid in acetone. The precipitated salt was sucked off and washed with diethyl ether. 2-(1*H*-imidazol-1-yl)ethyl retinoate ethanedioate (2:1) was obtained as an amorphous substance.

The invention also includes pharmaceutical preparations comprising 1*H*-imidazole-1-ethanol or an ester with formula I as the active agent. The invention particularly includes preparations suitable for topical application such as lotions, gels, creams and sticks. In these preparations 0.5—10% of active substance is preferably applied. The preparations may be prepared with the aid of methods commonly used in pharmacy. A lotion may for instance be obtained by dissolving the active substance in a mixture of water and 2-propanol or ethanol and mixing the solution obtained with a thickening agent such hydroxypropylcellulose or acrylic acid polymer (for instance Carbopol 940). In the end product the amount of alcohol can vary from 10 to 80%. The amount of thickening agent depends from the desired viscosity and may be for hydroxypropylcellulose 0.1—2% and for acrylic acid polymer 0.1—1%. With a low alcohol content (≤ 30%) also other thickening agents may be applied, for instance hydroxyethylcellulose, hydroxyethylmethylcellulose, hydroxypropylmethylcellulose, methylcellulose or sodium carboxymethylcellulose. Under certain conditions alginates, vegetable gums and starch derivatives are also applicable as thickening agents. If desired, penetration promoting agents and/or humidity controlling agents may be applied to the lotions such as isopropyl myristate, dioctyl sebacate, propylene glycol, glycerol in concentrations up to 10%. A gel may be prepared by mixing an alcohol such as 2-propanol or ethanol with a gel forming agent and dissolving the active substance in the mixture. Suitable gel forming agents are polyacrylic acid (for instance 0.5—1.5% Carbopol 949, 934 or 941), hydroxypropylcellulose (for instance 2—3% Klucel HF), cellulosederivatives, alginates, vegetable gums and starch derivatives. The amount of alcohol may vary from 10 to 80%. Additional constituents may be added to the gel which may serve as penetration promoting agents and as plasticizer of the film remaining on the skin. Examples of such additions are: isopropyl myristate, ethyl lactate, dioctylsebacate, dioctyl phtalate, glyceryl triacetate, polyethylene glycol, propylene glycol, glycerol.

These substances and many others with similar properties may be added in concentrations of 0.5—10% by weight.

A cream may be prepared by melting one or more fatty substances with a emulsifying agent and mixing the melt with a solution of the active substance in a mixture of water and an organic solvent and cooling the mixture obtained under thorough mixing to room temperature. As fatty substances may be applied animal or synthetic fatty alcohols such as cetostearyl alcohol, liquid paraffins, vegetable, animal, mineral and synthetic oils and fats and vegetable, animal and synthetic waxes.

A suitable emulsifying agent is polyoxyethylenecetyl ether. Also other emulsifying agents or combinations thereof may be used, preferably from the group of non-ionogenic emulsifying agents. Suitable organic solvents include propylene glycol, ethanol, isopropanol, butanediol-1,3, Solketal and glycerin formal.

Sticks may be prepared by mixing a solution of the active substance in a higher alcohol, for instance octyldodecanol, with a melt of waxy, oily substances and allowing the mixture to cool in moulds.

Examples of applicable substances are: waxy substances: bees wax, cetyl palmitate, cetyl alcohol, Cutina LM, carnauba wax, candelilla wax, microcrystalline paraffin, cetostearyl alcohol, PCL-solid, stearic acid, palmitic acid, glycerin monostearate, wool wax alcohols; oily substances: octyl dodecanol, isopropyl myristate and palmitate, ricinus oil, olive oil, Miglyol 812, oleyl alcohol, dioctyl sebacate, liquid paraffin.

Also fatty substances as vaselin, theobroma oil and wool wax may be applied.

Also some alcohols may be added which may serve as solvent for the active substance and/or penetration promoting agents, such as propylene glycol, butanediol-1.3 and hexylene glycol.

The pH of the preparations may be brought to the desired value (for instance 6) by addition of an organic or inorganic acid such as citric acid, tartaric acid, lactic acid, gluconic acid, hydrochloric acid and phosphoric acid.

The percentages mentioned are always percents by weight in the end product.

In the following examples the preparation of pharmaceutical compositions according to the invention is described.

### Example 9 (Lotion)

3 Parts by weight of 2-(1*H*-imidazol-1-yl)ethyl benzoate are dissolved in a mixture of 50 parts by weight of 2-propanol and 40 parts by weight of water. To this was added 0.5 part by weight of isopropyl myristate. Subsequently the liquid was thickened by addition of 0.5 part by weight of hydroxypropylcellulose. The pH was brought to 6 by addition of citric acid and the liquid was made up with water to 100 parts by weight.

### Example 10 (Gel)

1 Part by weight of polyacrylic acid (Carbopol 940) was added to a mixture of 50 parts by weight of 2-propanol and 45 parts by weight of water. After complete dispersion 2 parts by weight of 2-(1*H*-imidazol-1-yl)ethyl benzoate were added under careful stirring and dissolved. The pH was brought to 6 with citric acid and water was added to 100 parts by weight.

### Example 11 (Cream)

6 Parts by weight of cetostearyl alcohol are melted at a temperature of about 70°C with 2.5 parts by weight of polyoxyethylenecetyl ether (Cetamacrogol 1000) and 5 parts by weight of liquid paraffin. This liquid is combined with a solution of 2 parts by weight of 2-(1*H*-imidazol-1-yl)ethyl benzoate in 10 parts by

weight of propylene glycol and 74.5 parts by weight of water of 70°C. The liquids are cooled to room temperature under thorough mixing and the pH is brought to 6 with citric acid.

Example 12 (Stick)

5 Parts by weight of cetyl alcohol, 30 parts by weight of bees wax, 20 parts by weight of cetyl palmitate and 17 parts by weight of liquid paraffin are melted together, 3 parts by weight of 2-(1*H*-imidazol-1-yl)ethyl benzoate are dissolved under gentle heating in 25 parts by weight of octyldodecanol, and the two liquids are mixed with each other. The mixture is poured into moulds and allowed to congeal at room temperature.

Carbopol 940, 934 and 941, Klucel HF, Solketal, Cutina LM, PCL-solid, Myglocyl 812 and Cetamacrogol 1000 are registered trade marks.

**Claims for the Contracting States: BE CH DE FR GB IT LI LU NL SE**

1. 1*H*-imidazole-1-ethanol and esters derived therefrom with the formula

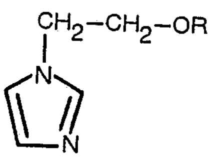

I

in which R represents an acyl residue of an organic carboxylic acid, which does not bear further carboxylic groups, either in the free or salt form, and their acid addition salts for use as sebum synthesis inhibiting agents.

2. Esters of the formula

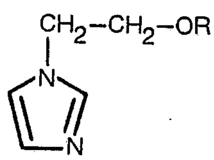

I

in which R is as defined in claim 1 with the exception of the acetic acid residue, and their acid addition salts.

3. Esters according. to claim 2, characterized in that R is the residue of benzoic acid, optionally substituted with one or more C1—C6 alkyl or alkoxy groups or chlorine, bromine or iodine atoms.

4. Esters according to claim 2, characterized in that R is the residue of a phenylalkanecarboxylic acid, in which the phenyl group may be substituted with C1—C6 alkyl or alkoxy groups or chlorine, bromine or iodine atoms and the alkane moiety contains at most four carbon atoms.

5. Esters according to claim 2, characterized in that R is a group ACO—, in which A is a sulphur, oxygen or nitrogen containing heterocyclic five- or six-membered ring.

6. Esters according to claim 2, characterized in that R is the residue of a aliphatic carboxylic acid.

7. 2-(1*H*-imidazol-1-yl)ethyl benzoate and its acid addition salts.

8. 2-(1*H*-imidazol-1-yl)ethyl benzenepropanoate and its acid addition salts.

9. 2-(1*H*-1-imidazol-1-yl)ethyl 2-thiophenecarboxylate and its acid addition salts.

10. 2-(1*H*-imidazol-1-yl)ethyl 2,2-dimethylpropanoate and its acid additions salts.

11. Process for the preparation of compounds according to claim 2, characterized in that 1*H*-imidazole-1-ethanol is reacted with an acid halide derived from R and the compound obtained is, if desired, converted into an acid addition salt.

12. Process according to claim 11, characterized in that the alcohol and the acid halide are heated in a polar solvent.

13. Process according to claim 11 or 12, characterized in that the reaction is carried out in the presence of an acid binding agent.

14. Process for the preparation of compounds according to claim 2, characterized in that imidazole is reacted with a compound of the formula

$$X—CH_2—CH_2—OR \qquad II$$

in which R is as defined in claim 2 and X represents a halogen atom, and the compound obtained is, if desired, converted into an acid addition salt.

15. Process according to claim 14, characterized in that imidazole and the compound of formula II are heated together without solvent.

16. Pharmaceutical preparations, characterized in that they comprise as the active constituent 1*H*-imidazol-1-ethanol or an ester derived therefrom as defined in claim 1.

**Claims for the Contracting State: AT**

1. Process for the preparation of esters of the formula

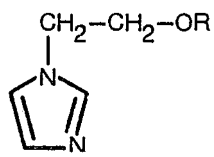

I

in which R represents an acyl residue of an organic carboxylic acid which does not bear further carboxylic groups, either in the free or salt form, with the exception of the acetic acid residue, and the acid addition salts of said esters, characterized in that 1*H*-imidazole-1-ethanol is reacted with an acid halide derived from R and the compound obtained is, if desired, converted into an acid addition salt.

2. Process according to claim 1, characterized in that the alcohol and the acid halide are heated in a polar solvent.

3. Process according to claim 1 or 2, characterized in that the reaction is carried out in the presence of an acid binding agent.

4. Process for the preparation of compounds defined in claim 1, characterized in that imidazole is reacted with a compound of the formula

$$X—CH_2—CH_2—OR \qquad II$$

in which R is as defined in claim 1 and X represents a halogen atom, and the compound obtained is, if desired, converted into an acid addition salt.

5. Process according to claim 4, characterized in that imidazole and the compound of formula II are heated together without solvent.

**Patentansprüche für die Vertragsstaaten: BE CH DE FR GB IT LI LU NL SE**

1. 1H-Imidazol-1-ethanol und davon abgeleitete Ester der Formel:

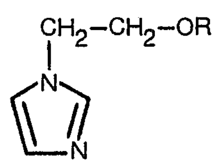

I

worin R einen Acylrest einer organischen Carbonsäure darstellt, der keine weiteren Carboxylgruppen, entweder in freier Form oder in Salzform, trägt, und deren Säureadditionssalze für die Verwendung als Sebumsyntheseinhibitoren.

2. Ester der Formel:

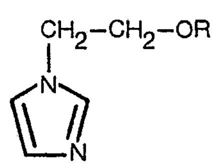

I

worin R wie in Anspruch 1 definiert ist, mit Ausnahme des Essigsäurerestes, und deren Säureadditionssalze.

3. Ester nach Anspruch 2, dadurch gekennzeichnet, dass R der Rest der Benzoesäure ist, der gegebenenfalls durch eine oder mehrere Alkyl- oder Alkoxygruppen mit 1 bis 6 Kohlenstoffatomen oder Chlor-, Brom- oder Jodatome substituiert ist.

4. Ester nach Anspruch 2, dadurch gekennzeichnet, dass R der Rest einer Phenylalkancarbonsäure ist, in dem die Phenylgruppe durch Alkyl- oder Alkoxygruppen mit 1 bis 6 Kohlenstoffatomen oder Chlor-, Brom- oder Jodatome substituiert sein kann und der Alkanrest höchstens 4 Kohlenstofatome enthält.

5. Ester nach Anspruch 2, dadurch gekennzeichnet, dass R eine Gruppe der Formel ACO- ist, worin A ein Schwefel, Sauerstoff oder Stickstoff enthaltender heterocyclischer 5- oder 6-gliedriger Ring ist.

6. Ester nach Anspruch 2, dadurch gekennzeichnet, dass R der Rest einer aliphatischen Carbonsäure ist.

7. 2-(1H-Imidazol-1-yl)-ethylbenzoat und dessen Säureadditionssalze.

8. 2-(1H-Imidazol-1-yl)-ethylbenzolpropanoat und dessen Säureadditionssalze.

9. 2-(1H-1-Imidazol-1-yl)-ethyl-2-thiophencarboxylat und dessen Säureadditionssalze.

10. 2-(1H-Imidazol-1-yl)-ethyl-2,2-dimethylpropanoat und dessen Säureadditionssalze.

11. Verfahren zur Herstellung von Verbindungen gemäss Anspruch 2, dadurch gekennzeichnet, dass man 1H-Imidazol-1-ethanol mit einem von R abgeleiteten Säurehalogenid umsetzt und die erhaltene Verbindung gewünschtenfalls in ein Säureadditionssalz überführt.

12. Verfahren nach Anspruch 11, dadurch gekennzeichnet, dass man den Alkohol und das Säurehalogenid in einem polaren Lösungsmittel erhitzt.

13. Verfahren nach Anspruch 11 oder 12, dadurch gekennzeichnet, dass man die Reaktion in Gegenwart eines säurebindenden Mittels ausführt.

14. Verfahren zur Herstellung von Verbindungen gemäss Anspruch 2, dadurch gekennzeichnet, dass man Imidazol mit einer Verbindung der Formel:

$$X—CH_2—CH_2—OR \qquad II$$

worin R wie in Aspruch 2 definiert ist und X ein Halogenatom darstellt, umsetzt und die erhaltene Verbindung gewünschtenfalls in ein Säureadditionssalz überführt.

15. Verfahren nach Anspruch 14, dadurch gekennzeichnet, dass man Imidazol und die Verbindung der Formel II zusammen ohne Lösungsmittel erhitzt.

16. Pharmazeutische Präparate, dadurch gekennzeichnet, dass sie als aktiven Bestandteil 1H-Imidazol-1-ethanol oder einen davon abgeleiteten Ester, wie er in Anspruch 1 definiert ist, aufweisen.

**Patentansprüche für der Vertragsstaat: AT**

1. Verfahren zur Herstellung von Estern der Formel:

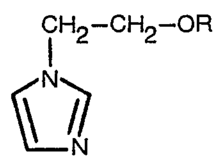

I

worin R einen Acylrest einer organischen Carbonsäure, der keine weiteren Carboxylgruppen, entweder in freier Form oder in Salzform, trägt, mit Ausnahme des Essigsäurerestes darstellt, und von Säureadditionssalzen der genannten Ester, dadurch gekennzeichnet, dass man 1H-Imidazol-1-ethanol mit einem von R abgeleiteten Säurehalogenid umsetzt und die erhaltene Verbindung gewünschtenfalls in ein Säureadditionssalz überführt.

2. Verfahren nach Anspruch 1, dadurch gekennzeichnet, dass man den Alkohol und das Säurehalogenid in einem polaren Lösungsmittel erhitzt.

3. Verfahren nach Anspruch 1 oder 2, dadurch gekennzeichnet, dass man die Reaktion in Gegenwart eines säurebindenden Mittels ausführt.

4. Verfahren zur Herstellung von in Anspruch 1 definierten Verbindungen, dadurch gekennzeichnet, dass man Imidazol mit einer Verbindung der Formel:

$$X—CH_2—CH_2—OR \qquad II$$

worin R wie in Anspruch 1 definiert ist und X ein Halogenatom darstellt, umsetzt und die erhaltene Verbindung gewünschtenfalls in ein Säureadditionssalz überführt.

5. Verfahren nach Anspruch 4, dadurch gekennzeichnet, dass man Imidazol und die Verbindung der Formel II zusammen ohne Lösungsmittel erhitzt.

**Revendications pour les Etats contractants: BE CH DE FR GB IT LI LU NL SE**

1. Le 1*H*-imidazole-1-éthanol et les esters qui en dérivent de formule

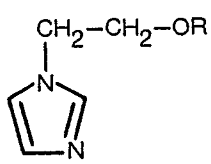

I

où R représente un résidu acyle d'un acide organique carboxylique qui ne porte pas de radicaux carboxyliques supplémentaires, sous la forme libre ou salifiée, et leurs sels d'addition d'acides à utiliser comme agents inhibant la synthèse du sébum.

2. Esters de formule

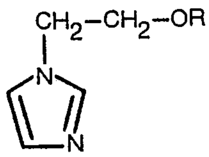

I

où R est tel que défini dans la revendication 1, à l'exception du résidu de l'acide acétique, et leurs sels d'addition d'acides.

3. Esters suivant la revendication 2, caractérisés en ce que R est le résidu de l'acide benzoïque, portant éventuellement comme substituants un ou plusieurs radicaux alcoyle ou alcoyle en C1—C6 ou atomes de chlore, de brome ou d'iode.

4. Esters suivant la revendication 2, caractérisés en ce que R est le résidu d'un acide phénylalcane-carboxylique dont le radical phényle peut porter comme substituants des radicaux alcoyle ou alcoxy en C1—C6 ou atomes de chlore, de brome ou d'iode et dont la partie alcane compte au maximum 4 atomes de carbone.

5. Esters suivant la revendication 2, caractérisés en ce que R est un radical ACO où A est un hétérocycle de 5 ou 6 chaînons contenant du soufre, de l'oxygène ou de l'azote.

6. Esters suivant la revendication 2, caractérisés en ce que R est le résidu d'un acide aliphatique carboxylique.

7. Le benzoate de 2-(1*H*-imidazol-1-yl)éthyle et ses sels d'addition d'acides.

8. Le benzènepropanoate de 2-(1*H*-imidazol-1-yl)éthyle et ses sels d'addition d'acides.

9. Le 2-thiophènecarboxylate de 2-(1*H*-1-imidazol-1-yl)éthyle et ses sels d'addition d'acides.

10. Le 2,2-diméthylpropanoate de 2-(1*H*-imidazol-1-yl)éthyle et ses sels d'addition d'acides.

11. Procédé de préparation de composés suivant la revendication 2, caractérisé en ce que le 1*H*-imidazole-1-éthanol est mis à réagir avec un halogénure d'acide issu de R et, si la chose est souhaitée, le composé résultant est converti en un sel d'addition d'acide.

12. Procédé suivant la revendication 11, caractérisé en ce que l'alcool et l'halogénure d'acide sont chauffés dans un solvant polaire.

13. Procédé suivant la revendication 11 ou 12, caractérisé en ce que la réaction est exécutée en présence d'un agent accepteur d'acide.

14. Procédé de préparation de composés suivant la revendication 2, caractérisé en ce que l'imidazole est mis à réagir avec un composé de formule

$$X—CH_2—CH_2—OR \qquad II$$

où R est tel que défini dans la revendication 2 et X représente un atome d'halogène, et, si la chose est souhaitée, le composé résultant est converti en un sel d'addition d'acide.

15. Procédé suivant la revendication 14, caractérisé en ce que l'imidazole et le composé de formule II sont chauffés ensemble sans solvant.

16. Préparations pharmaceutiques, caractérisées en ce qu'elles comprennent comme constituant actif du 1*H*-imidazole-1-éthanol ou un ester qui en dérive tel que défini dans la revendication 1.

**Revendications pour l'Etat contractant: AT**

1. Procédé de préparation d'esters de formule

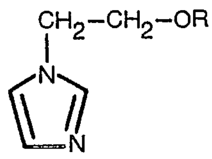

I

où R représente un résidu acyle d'un acide organique carboxylique qui ne porte pas de radicaux carboxyliques supplémentaires, sous la forme libre ou salifiée, à l'exception du résidu de l'acide acétique, et des sels d'addition d'acides de ces esters, caractérisé en ce que le 1*H*-imidazole-1-éthanol est mis à réagir avec un halogénure d'acide issu de R et, si la chose est souhaitée, le composé résultant est converti en un sel d'addition d'acide.

2. Procédé suivant la revendication 1, caractérisé en ce l'alcool et l'halogénure d'acide sont chauffés dans un solvant polaire.

3. Procédé suivant la revendication 1 ou 2, caractérisé en ce que la réaction est exécutée en présence d'un agent accepteur d'acide.

4. Procédé de préparation de composés suivant la revendication 1, caractérisé en ce que l'imidazole est mis à réagir avec un composé de formule

$$X—CH_2—CH_2—OR \qquad II$$

où R est tel que défini dans la revendication 1 et X représente un atome d'halogène, et, si la chose est souhaitée, le composé résultant est converti en un sel d'addition d'acide.

5. Procédé suivant la revendication 4, caractérisé en ce que l'imidazole et le composé de formule II sont chauffés ensemble sans solvant.